# NOUVEAU FASCICULE DE BREVET EUROPEEN

(11) **EP 1 800 650 B2**
(45) Date de publication et mention de la décision concernant l'opposition: **14.08.2013**
(45) Mention de la délivrance du brevet: 03.11.2010
(21) Numéro de dépôt: 06301271.0
(22) Date de dépôt: 20.12.2006
(51) Int. Cl.: A61K 8/34, A61K 8/37, A61Q 1/04, A61Q 19/00

(54) **Composition cosmétique à effet volumateur**
Kosmetische Zusammensetzung zur Bereitstellung eines Volumeneffekts
Cosmetic composition providing a voluminous effect

(30) Priorité: 21.12.2005 FR 0513075
(43) Date de publication de la demande: 27.06.2007
(73) Titulaire: L'Oréal, 75008 Paris (FR)
(72) Inventeur: Blin, Xavier, 75015, PARIS (FR); Barba, Claudia, 75009 PARIS (FR)
(74) Mandataire: Le Coupanec, Pascale A.M.P.

(56) Documents cités:
- EP-A1- 1 238 651
- EP-A1- 1 283 032
- WO-A-2004/030605
- WO-A1-2005//060922
- DE-A1- 19 541 967
- US-A- 4 002 775
- US-A- 4 659 562
- US-A- 5 098 694
- US-A1- 2002 028 223
- US-A1- 2005 036 960
- US-A1- 2005 196 347
- US-B1- 6 403 110
- Brochure pour Lexgard GMCy
- PCPC base de données pour glyceryl laurate
- PCPC base de données pour glyceryl myristate
- PCPC base de données pour polysorbate 20
- PCPC base de données pour PEG-4 Laurate
- PCPC base de données pour polyglyceryl-4 isostearate
- PCPC base de données pour polysorbate 60

## Description

L'invention concerne le maquillage naturel de la peau et/ou des lèvres et vise plus particulièrement à renforcer leur carnation naturelle et, pour ce qui est des lèvres, à leur conférer un effet volumateur.

Par « maquillage naturel » de la peau, on entend selon l'invention un moyen permettant de colorer naturellement la peau, par opposition aux moyens existants destinés à colorer artificiellement la peau à l'aide d'agents autobronzants ou d'agents stimulateurs de la synthèse de mélanine (pigmentation) et/ou d'agents de maquillage tels que des colorants, des pigments ou charges spécifiques aptes à conférer un effet optique de coloration de la peau.

Par « maquillage naturel » des lèvres, on entend selon l'invention un moyen permettant de colorer et/ou de repulper naturellement les lèvres, par opposition au maquillage classique des lèvres qui met en oeuvre uniquement des agents de maquillage tels que des colorants, des pigments spécifiques ou des particules réfléchissantes aptes à conférer un effet optique de coloration et/ou de volume (effet de plump) des lèvres.

Par « colorer naturellement », on entend selon l'invention stimuler la coloration naturellement rosée de la peau et/ou des lèvres.

Par « repulper », on entend selon l'invention augmenter la taille et/ou le volume et/ou l'épaisseur des lèvres et/ou les remodeler et/ou les lisser et/ou leur donner un aspect plus gonflé ou charnu.

Concernant ce dernier effet, il est manifeste qu'il répond à une demande permanente d'un grand nombre de consommatrices qui n'hésitent pas dans certains cas à recourir à la chirurgie esthétique pour obtenir un tel effet, qui devient alors permanent. Toutefois, cette irréversibilité, associée au risque de toxicité et/ou d'allergie accentué en raison du caractère invasif de l'intervention correspondante, peut être par ailleurs de nature dissuasive à l'égard d'autres consommatrices potentielles.

Plus accessible et non invasif, le maquillage conventionnel permet de mimer cet effet en jouant notamment sur des phénomènes d'optique qui consistent à créer de la brillance localisée dans des régions bien définies des lèvres ou bien en superposant deux types de brillance, de type ponctuel et diffus afin de tromper le regard et de créer ainsi une sensation de volume. Toutefois, cette alternative relève d'un effet virtuel, parfois non perceptible par la consommatrice.

Un des aspects de la présente invention vise précisément à obtenir des compositions cosmétiques aptes à procurer un réel effet « plump » en termes de taille et/ou de volume des lèvres.

En conséquence, selon l'un de ses aspects, l'invention concerne l'utilisation cosmétique d'au moins un glycol de chaîne hydrocarbonée en C₄-C₁₆ et/ou d'un ester hydroxylé résultant de l'estérification de polyol et d'acide(s) carboxylique(s) en C₄ à C₁₆ dans une composition de soin et/ou de maquillage de la peau et/ou des lèvres comme agent destiné à renforcer la carnation naturelle de la peau et/ou des lèvres et/ou le volume naturel des lèvres.

Selon un mode de réalisation particulier, ledit agent est un ester hydroxylé résultant de l'estérification de polyol et d'acide(s) carboxylique(s) en C₄ à C₁₆.

Selon un autre mode de réalisation particulier, ledit agent est un glycol de chaîne hydrocarbonée en C₆-C₁₄.

Selon encore un autre mode de réalisation particulier, ledit agent est un glycol de chaîne hydrocarbonée en C₄ à C₁₆ et un ester hydroxylé résultant de l'estérification de polyol et d'acide carboxylique en C₄ à C₁₆. Par utilisation cosmétique, on entend une utilisation non thérapeutique susceptible de produire un effet sans pour autant prévenir ou corriger un dysfonctionnement des matières kératiniques.

Par utilisation cosmétique, on entend également une utilisation d'une composition contenant des ingrédients physiologiquement acceptables.

Selon un autre de ses aspects, la présente invention vise également des compositions cosmétiques destinées au soin et/ou au maquillage de la peau et/ou des lèvres contenant dans un milieu physiologiquement acceptable, au moins :
- une matière colorante et
- un glycol de chaîne hydrocarbonée en C₄ à C₁₆ et un ester hydroxylé de polyol et d'acide(s) carboxylique(s) en C₄ à C₁₆.

Selon un autre de ses aspects, la présente invention est une composition cosmétique destinée au maquillage de la peau et/ou des lèvres contenant, dans un milieu physiologiquement acceptable, moins de 5 % en poids d'eau et au moins un glycol de chaîne hydrocarbonée en C₄-C₁₆, et comprenant de plus un ester hydroxylé de polyol et d'acide(s) carboxylique(s) en C₄-C₁₆.

Selon un mode de réalisation, la présente invention vise une composition cosmétique destinée au maquillage de la peau et/ou des lèvres contenant dans un milieu physiologiquement acceptable, au moins :
- une matière colorante, et
- au moins un glycol de chaîne hydrocarbonée en C₅-C₇ ou C₉-C₁₆ et un ester hydroxylé de polyol et d'acide(s) carboxylique(s) en C₄ à C₁₆.

Selon un mode de réalisation, la présente invention vise une composition cosmétique destinée au soin de la peau et/ou des lèvres contenant dans un milieu physiologiquement acceptable, au moins :
- une matière colorante,
- au moins un glycol de chaîne hydrocarbonée en C₅-C₇ ou C₉-C₁₆ et un ester hydroxylé de polyol et d'acide(s) carboxylique(s) en C₄ à C₁₁ ou C₁₃ à C₁₆.

Selon un autre de ses aspects, l'invention porte sur des compositions cosmétiques destinées au soin et/ou maquillage de la peau et/ou des lèvres contenant, dans un milieu physiologiquement acceptable :
- au moins un glycol de chaîne hydrocarbonée en C₄ à C₁₆ et un ester hydroxylé de polyol et d'acide(s) carboxylique(s) en C₄ à C₁₆, et
- moins de 5 % en poids d'eau par rapport à son poids total.

Les compositions cosmétiques selon l'invention sont plus particulièrement destinées à une application topique.

Un autre aspect de l'invention concerne un procédé de maquillage de la peau et/ou les lèvres, visant à donner un effet bonne mine au teint mettant en oeuvre une composition conforme à l'invention ainsi qu'un procédé de soin et/ou de maquillage des lèvres comprenant l'application sur les lèvres d'une composition également conforme à la présente invention.

Les compositions selon l'invention peuvent également être utilisées pour avoir naturellement bonne mine, en application en particulier au niveau du visage. Les compositions peuvent être appliquées quotidiennement sur l'ensemble du visage pour obtenir un teint naturel homogène.

L'application peut également être limitée, renouvelée ou renforcée au niveau des joues et des pommettes par exemple pour accentuer l'effet bonne mine sur des zones particulières du visage.

Ainsi, les compositions peuvent être sous la forme d'une base de soin pour la peau, d'une crème de soin (crème de jour, de nuit, anti-rides), d'une base de maquillage, ou d'une crème de soin teintée.

Les compositions selon l'invention peuvent également être utilisées pour dissimuler ou estomper les défauts de la peau, en particulier les poches et/ou les cernes péri-oculaires, pour obtenir un teint uniforme, homogène, d'aspect naturel, lumineux, vivant. Elles peuvent ainsi permettre d'homogénéiser et/ou clarifier le teint et/ou diminuer l'effet de teint brouillé.

Les compositions peuvent également être utilisées pour améliorer l'aspect des lèvres ou le contour des lèvres, notamment pour modifier la coloration des lèvres ou stimuler leur coloration naturelle, pour augmenter le volume des lèvres et/ou à les modeler et/ou à les rendre plus lisses. Elles peuvent être utilisées pour repulper les lèvres, notamment en augmentant la taille et/ou le volume et/ou l'épaisseur des lèvres et/ou les remodeler et/ou les lisser et/ou leur donner un aspect plus gonflé ou charnu.

Selon les sites d'application auxquels elles sont dédiées, les compositions peuvent être un produit de maquillage naturel de la peau sous forme d'une base ou crème de soin pour la peau, d'une base de maquillage, d'une crème teintée, d'un fond de teint sous forme liquide ou semi-solide ou de poudre, d'un soin de contour de l'oeil, un sérum de soin anti-cernes, d'un stick anti-cernes ou d'un produit de maquillage naturel des lèvres sous la forme d'un rouge à lèvres, d'un gloss liquide, d'une pâte de rouges à lèvres, d'un crayon pour le contour des lèvres, d'un baume ou soin des lèvres, d'un vernis à lèvres autrement nommé laque à lèvres.

Certains des esters considérés selon l'invention sont déjà connus pour leurs propriétés antiseptiques et classiquement mis en oeuvre à ce titre dans des compositions destinées à être administrées à l'homme ou l'animal comme des compositions pharmaceutiques, alimentaires et cosmétiques. La présence de ces esters garantit une stabilité dans le temps aux compositions correspondantes. Ainsi le document US 5 690 919 décrit des déodorants comprenant du monocaprylate de glycéride à titre d'anti-microbien. De même, le document DE 1 9541 967 décrit des lotions aqueuses comprenant ces mêmes esters à titre de microbicides. De manière plus générale, le brevet US 4 002 775 propose d'utiliser des monosters de polyol et d'acide gras aliphatique en C₁₂ à titre d'agent microbicide dans des compositions dédiées à l'alimentation.

Pour sa part, l'invention résulte plus particulièrement de la mise en évidence par les inventeurs que les composés considérés selon l'invention s'avèrent contre toute attente posséder des propriétés avantageuses en termes de maquillage naturel de la peau et/ou des lèvres.

Ainsi, ce qui concerne plus particulièrement les lèvres, il a été noté que la mise en contact des lèvres avec ces composés induisait un effet de gonflement significatif apte à procurer l'effet repulpeur recherché.

### GLYCOLS

Le glycol mis en oeuvre selon l'invention peut posséder une chaîne hydrocarbonée en C₄-C₁₆ et a de préférence une chaîne hydrocarbonée en C₆-C₁₄, en C₇-C₁₂ et encore mieux en C₇ à C₁₀.

Selon un mode de réalisation particulier, le glycol mis en oeuvre selon l'invention possède une chaîne hydrocarbonée en C₅-C₇ ou C₉-C₁₆, ou encore C₆-C₇ ou C₁₀-C₁₆.Dans le mode de réalisation, où le glycol est utilisé sans ester de polyol, il est avantageux d'utiliser un glycol de chaîne hydrocarbonée en C₆-C₁₄.

Par glycol de chaîne hydrocarboné en Cₓ, on entend désigner les composés de formule (I) : 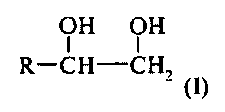 dans laquelle R est un radical alkyle en Cₓ₋₂.

Le glycol de chaîne hydrocarboné peut avoir, une chaîne hydrocarbonée en C₈-C₁₀.

Il peut notamment s'agir de caprylyl glycol et notamment, le composé commercialisé sous la dénomination CAPRYLYL GLYCOL DERMOSOFT OCTIOL de chez STRAETMANS.

Le ou les glycols peuvent être présents dans ces compositions à une teneur variant de 0,05 à 20 %, notamment de 0,1 à 10 %, voire moins de 5 % en poids, en particulier de 0,1 à 5 %, et plus particulièrement 0,5 à 2 % en poids du poids total de la composition.

### ESTERS

Les esters plus particulièrement considérés selon la présente invention sont des esters hydroxylés résultant de l'estérification de polyol et d'acide carboxylique(s) en C₄ à C₁₆, et plus particulièrement en C₆ à C₁₂, notamment en C₇ à C₁₀, et plus particulièrement en C₈ à C₉.

Selon un mode particulier de l'invention, les esters mis en oeuvre peuvent résulter de l'estérification de polyol et d'acide carboxylique(s) en C₄ à C₉ ou en C₁₁ ou en C₁₃ à C₁₆.

Selon un mode particulier de l'invention, les esters mis en oeuvre peuvent résulter de l'estérification de polyol et d'acide carboxylique(s) en C₄ à C₁₁ ou en C₁₃ à C₁₆.

Les esters utilisés selon l'invention sont sous une forme hydroxylée, c'est-à-dire portent au moins une fonction hydroxyle, de préférence 2, voire 3 ou plus, les fonctions hydroxylées étant présentes sur le résidu alcool de l'ester.

Avantageusement, les esters sont en C₁₀ à C₂₀ et possèdent au moins une chaîne grasse.

D'une manière générale, ils dérivent de l'estérification d'au moins une fonction hydroxyle d'un polyol par un acide carboxylique en C₄ à C₁₆.

Selon un mode de réalisation particulier, les esters convenant à la présente invention peuvent dériver de l'estérification d'un polyol par différents acides carboxyliques sous réserve bien sûr que l'ester ainsi obtenu possède au moins une et, de préférence, deux fonctions hydroxyles libres. Il peut s'agir d'un mono-ester hydroxylé, d'un di-ester hydroxylé ou d'un de leurs mélanges.

### Polyols

Par polyol, au sens de l'invention, on entend tout molécule organique comportant dans sa structure chimique au moins deux groupements hydroxyle (OH).

Le polyol peut être en particulier un composé hydrocarboné, linéaire, ramifié ou cyclique, saturé ou insaturé portant au moins deux, et en particulier au moins trois fonctions OH.

Le polyol peut être en particulier un composé hydrocarboné comportant au moins 2 atomes de carbone, de préférence moins de 15 atomes de carbone, et portant au moins deux groupes hydroxyle, de préférence de 2 à 10 groupes hydroxyle.

De préférence, il s'agit d'un composé hydrocarboné ayant de 2 à 12 atomes de carbone, et encore plus préférentiellement de 2 à 8 atomes de carbone.

Le polyol peut être un composé ayant de 2 à 8 atomes de carbone et de 2 à 6 fonctions hydroxyles, tel que, par exemple, l'éthylène glycol, le glycérol, le 1,2,3-trihydroxyhexane, le butane diol, le propane-1,2 diol, l'érythritol, l'arabitol, l'adonitol, et le dulcitol, les pentanediols et en particulier le 1,2-pentanediol, le sorbitol ou leurs mélanges.

Des dérivés du glycérol sont par exemple le butyldiglycol, le polyglycéryl-3-diisostéarate, et l'huile de ricin. Le polyol peut être choisi parmi les polymères et les copolymères de glycérol, comme par exemple l'hexaglycérol et le diglycérol.

Des dérivés du glycol sont par exemple l'éthylène glycol, le propylène glycol, l'hexylène glycol, l'isoprène glycol, le butylène glycol, et le pentylène glycol et ceux définis précédemment.

Le polyol peut également être choisi parmi les sucres tels que le glucose, le fructose, le xylose, le tréhalose, le saccharose, le maltose, le lactose, et leurs mélanges.

On peut également employer un mélange de polyols.

Le polyol utilisé selon l'invention peut être, plus particulièrement, choisi parmi le glycérol, les glycols et leurs dérivés.

Les polyols particulièrement préférés sont choisis parmi la glycérine, le 1,2-propylène glycol, ou un mélange de deux ou plusieurs de ces polyols.

### Acide carboxylique

L'acide carboxylique peut être linéaire ou ramifié, saturé ou insaturé.

Avantageusement, il s'agit d'un acide monocarboxylique, linéaire.

A titre illustratif d'exemples d'acide monocarboxylique convenant à l'invention, on peut notamment citer l'acide butanoïque, l'acide pentanoïque, l'acide héxanoïque, l'acide héptanoïque, l'acide octanoïque, l'acide nonanoïque, l'acide décanoïque, l'acide undécanoïque, l'acide dodécanoïque, l'acide tridécanoïque, l'acide tétradécanoïque, l'acide héptadécanoïque, l'acide héxadécanoïque, l'acide pentadécanoïque.

A titre représentatif des acides ramifiés, on peut plus particulièrement citer l'acide isobutanoïque, l'acide isopentanoïque, l'acide pivalique, l'acide isohéxanoïque, l'acide isohéptanoïque, l'acide isooctanoïque, l'acide diméthyloctanoïque, l'acide isononanoïque, l'acide isodécanoïque, l'acide isoundécanoïque, l'acide isododécanoïque, l'acide isotridécanoïque, l'acide isotétradécanoïque, l'acide isopentadécanoïque, l'acide isohéxadécanoïque, l'acide 2-éthylhéxanoïque, l'acide 2-butyloctanoïque, l'acide 2-héxildécanoïque.

Conviennent également à la présente invention les hydroxyacides tels que l'acide 2-hydroxybutanoïque, l'acide 2-hydropentanoïque, l'acide 2-hydroxyhéxanoïque, l'acide 2-hydroxyhéptanoïque, l'acide 2-hydroxyoctanoïque, l'acide 2-hydroxynonanoïque, l'acide 2-hydroxydécanoïque, l'acide 2-hydroxyundécanoïque, l'acide 2-hydroxydodécanoïque, l'acide 2-hydroxytridécanoïque, l'acide 2-hydroxytétradécanoïque, et l'acide 2-hydroxyhéxadécanoïque.

Il s'agit plus particulièrement d'un acide non-hydroxylé en C₇ à C₁₀ et plus particulièrement de l'acide héptanoïque, de l'acide caprylique et de l'acide caprique.

Conviennent tout particulièrement à l'invention les esters choisis parmi les caprylate de mono- et /ou di-glycéryle, hèptanoate de mono- et/ou di-glycéryle, caprylate de propylèneglycol, héptanoate de propylèneglycol et leurs mélanges.

Il s'agit plus particulièrement du caprylate de monoglycéryle et de ses mélanges.

On citera notamment les composés commercialisés sous la dénomination CAPMUL MCM ou AKOLINE MCM (Caprylate/Caprate de glyceryle) de chez ABITEC, ou le DERMOSOFT GMCY (Caprylate de glycérol) de chez STRAETMANS, le CAPMUL 708 G (glyceryl caprylate à 75% de monoesters) de chez ABITEC et encore le CAPMUL 907P (propylène glycol heptanoate) de chez ABITEC ou encore CAPMUL 908P (propylène glycol Caprylate) de chez ABITEC.

Le ou les esters peuvent être présents dans ces compositions à une teneur variant de 0,05 à 20 %, notamment de 0,1 à 10 %, voire moins de 5 % en poids, en particulier de 0,2 à 5 %, et plus particulièrement 0,5 à 2 % en poids du poids total de la composition.

Les esters peuvent être introduits dans les compositions cosmétiques conformes à la présente invention selon des protocoles conventionnels.

Quelque soit sa nature, l'agent destiné à renforcer la carnation naturelle de la peau et/ou des lèvres et/ou le volume naturel des lèvres peut être présent dans ces compositions à une teneur variant de 0,05 à 20 %, notamment de 0,1 à 10 %, voire moins de 5 % en poids, en particulier de 0,2 à 5 %, et plus particulièrement 0,5 à 2 % en poids du poids total de la composition.

La composition de l'invention peut se présenter sous la forme solide, pâteuse ou liquide plus ou moins fluide. Elle peut être un gel anhydre, solide ou souple, une phase huileuse liquide, une mousse.

Selon une variante de l'invention, les compositions possèdent moins de 5% en poids, en particulier moins de 3% en poids, plus particulièrement moins de 1% en poids d'eau voire sont exemptes d'eau, c'est-à-dire anhydres.

### PHASE SOLVANT

Généralement, la composition selon l'invention comprend au moins une phase solvant non aqueuse.

Cette phase est susceptible de former une phase continue et contient, comme son nom l'indique, au moins un solvant organique non aqueux qui est de préférence un composé non soluble dans l'eau et liquide à température ambiante et pression atmosphérique.

Par « composé volatil », on entend, au sens de l'invention, tout composé (ou milieu non aqueux) susceptible de s'évaporer au contact des matières kératiniques ou les lèvres en moins d'une heure, à température ambiante et pression atmosphérique. Le composé volatil est un composé cosmétique volatil, liquide à température ambiante, ayant notamment une pression de vapeur non nulle, à température ambiante et pression atmosphérique, notamment ayant une pression de vapeur allant de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

Par opposition, on entend par « composé non volatil », un composé restant sur les matières kératiniques ou les lèvres à température ambiante et pression atmosphérique, au moins plusieurs heures et ayant notamment une pression de vapeur inférieure à 10⁻³ mm de Hg (0,13Pa).

Le composé volatil, non soluble dans l'eau et liquide à température ambiante est en particulier une huile (corps gras liquide à 25°c et pression atmosphérique) ou un solvant organique cosmétiquement acceptable. Par l'expression « cosmétiquement acceptable », on entend un composé dont l'utilisation est compatible avec une application sur les matières kératiniques.

Les huiles volatiles peuvent être des huiles hydrocarbonées, des huiles siliconées, des huiles fluorées ou leurs mélanges.

On entend par « huile hydrocarbonée », une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux « d'Isopars^{®} » ou de « Permetyls^{®} », les esters ramifiés en C₈-C₁₆, le néopentanoate d'iso-hexyle, et leurs mélanges. D'autres huiles hydrocarbonées volatiles comme les distillats de pétrole, notamment ceux vendus sous la dénomination « Shell Solt^{®} » par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité inférieure à 8 centistokes, et ayant notamment de 2 à 10 atomes de silicium, ces silicones comportant éventuellement des groupes alkyles ou alkoxy ayant de 1 à 22 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

La phase solvant non aqueuse peut également comprendre au moins un composé non volatil, non soluble dans l'eau et liquide à température ambiante, notamment au moins une huile non volatile, qui peut être en particulier choisie parmi les huiles hydrocarbonées et/ou siliconées et/ou fluorées non volatiles et de préférence brillantes

Comme huile hydrocarbonée non volatile, on peut notamment citer :
- les huiles hydrocarbonées d'origine végétale telles que les triglycérides constitués d'esters d'acides gras et de glycérol dont les acides gras peuvent avoir des longueurs de chaînes variées de C₄ à C₂₄, ces derniers pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations de « Miglyol 810® » , « 812® » et « 818® » par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges,
- les esters de synthèse comme les huiles de formule R1COOR2 dans laquelle R1 représente le reste d'un acide gras linéaire ou ramifié comportant de 1 à 40 atomes de carbone et R2 représente une chaîne hydrocarbonée notamment ramifiée contenant de 1 à 40 atomes de carbone à condition que R1 + R2 soit ≥ 10, comme par exemple l'huile de Purcellin (octanoate de cétostéaryle), le myristate d'isopropyle, le palmitate d'isopropyle, le benzoate d'alcool en C₁₂ à C₁₅, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique et leurs mélanges.

Les huiles de silicone non volatiles utilisables selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans la composition de l'invention sont notamment des huiles fluorosiliconées, des polyéthers fluorés, des silicones fluorées telles que décrit dans le document EP-A-847752.

### AGENT EPAISSISSANT/STRUCTURANT

Les compositions selon l'invention peuvent se présenter sous forme épaissie anhydre, par exemple sous la forme d'un stick notamment lorsqu'elle est dédiée à une application sur les lèvres. Elles peuvent être épaissies avec au moins un agent épaississant choisi parmi les gélifiants de phase grasse, les cires, les corps gras pâteux, les charges et leurs mélanges.

Comme gélifiant de phase grasse, on peut citer les argiles éventuellement modifiées comme les hectorites modifiées par un chlorure d'ammonium d'acide gras en C₁₀ à C₂₂, comme l'hectorite modifiée par du chlorure de di-stéaryl di-méthyl ammonium ; la silice ; les organopolysiloxanes élastomériques partiellement ou totalement réticulés, de structure tridimensionnelle, comme ceux commercialisés sous les noms KSG6, KSG16, KSG18 de Shin-Etsu, Trefil E-505C ou Trefil E-506C de Dow-Corning, Gransil SR-CYC, SR DMF10, SR-DC556, SR 5CYC gel, SR DMF 10 gel, SR DC 556 gel de Grant Industries, SF 1204 et JK 113 de Général Electric ; les galactommananes comportant un à six et mieux de deux à quatre groupes hydroxyle par ose, substitués par une chaîne alkyle saturée ou non, comme la gomme de guar alkylée par des chaînes alkyle en C₁ à C₆ et mieux en C₁ à C₃ et plus particulièrement la guar éthylée ayant un degré de substitution de 2 à 3 telle que celle vendue par la société Aqualon sous le nom N-HANCE-AG ; les gommes notamment siliconées comme les polydiméthylsiloxane PDMS ayant une viscosité > 500 000 centistokes et/ou un poids moléculaire supérieur ou égal à 200 000g/mol.

On peut également utiliser les polyamides siliconés du type polyorganosiloxane tels que ceux décrits dans les documents US-A-5,874,069, US-A-5,919,441, US-A-6,051,216 et US-A-5,981,680.

Ces polymères siliconés peuvent appartenir aux deux familles suivantes :
- des polyorganosiloxanes comportant au moins deux groupes capables d'établir des interactions hydrogène, ces deux groupes étant situés dans la chaîne du polymère, et/ou
- des polyorganosiloxanes comportant au moins deux groupes capables d'établir des interactions hydrogène, ces deux groupes étant situés sur des greffons ou ramifications.

Ces gélifiants sont utilisés par exemple à des concentrations de 0,2 à 15 % du poids total de la composition.

Les compositions peuvent contenir au moins une cire.

Par « cire » au sens de la présente invention, on entend un composé gras lipophile, solide à température ambiante (25 °C), à changement d'état solide/liquide réversible, ayant une température de fusion supérieure à 30 °C pouvant aller jusqu'à 200 °C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les cires utilisables dans l'invention sont des composés solides à température ambiante, destinés à structurer la composition en particulier sous forme de stick ; elles peuvent être hydrocarbonées, fluorées et/ou siliconées et être d'origine végétale, minérale, animale et/ou synthétique. En particulier, elles présentent une température de fusion supérieure à 40 °C et mieux supérieure à 45 °C.

Comme cire utilisable dans l'invention, on peut citer celles généralement utilisées dans le domaine cosmétique : elles sont notamment d'origine naturelle comme la cire d'abeilles, la cire de Carnauba, de Candelilla, d'Ouricoury, du Japon, de fibres de liège ou de canne à sucre, de riz, de Montan, la paraffine, les cires de lignite ou microcristalline, la cérésine ou l'ozokérite, les huiles hydrogénées comme l'huile de jojoba ; les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 40 °C et mieux à 45 °C, les cires de silicones comme les alkyl- ou alkoxydiméthicones ayant une chaîne alkyle ou alcoxy de 10 à 45 atomes de carbone, les esters de poly(di)méthylsiloxane solide à 40 °C dont la chaîne ester comporte au moins 10 atomes de carbone ; et leurs mélanges.

Les compositions selon l'invention contiennent avantageusement de la cire de polyéthylène de masse moléculaire en poids comprise entre 300 et 700, notamment égale à 500 g/mol.

A titre indicatif, la cire peut représenter de 0,01 à 50 %, de préférence de 2 à 40 %, et mieux de 5 à 30 % du poids total de la composition.

Les compositions peuvent également contenir au moins un composé pâteux.

Par « pâteux » au sens de la présente invention, on entend désigner un composé gras lipophile, à changement d'état solide/liquide réversible, et comportant à la température de 23 °C une fraction liquide et une fraction solide. On entend également par « pâteux », le polylaurate de vinyle.

Le composé pâteux est avantageusement choisi parmi :
- la lanoline et ses dérivés,
- les composés fluorés polymères ou non,
- les composés siliconés polymères ou non,
- les polymères vinyliques, notamment:
- les homopolymères d'oléfines
- les copolymères d'oléfines
- les homopolymères et copolymères de diènes hydrogénés
- les oligomères linéaires ou ramifiés, homo ou copolymères de (méth)acrylates d'alkyles ayant de préférence un groupement alkyle en C₈-C₃₀
- les oligomères homo et copolymères d'esters vinyliques ayant des groupements alkyles en C₈-C₃₀
- les oligomères homo et copolymères de vinyléthers ayant des groupements alkyles en C₈-C₃₀,
- les polyéthers liposolubles résultant de la polyéthérification entre un ou plusieurs diols en C₂-C₁₀₀, de préférence en C₂-C₅₀,
- les esters, et
- leurs mélanges.

Parmi les polyéthers liposolubles, on préfère en particulier les copolymères d'éthylène-oxyde et/ou de propylène-oxyde avec des alkylènes-oxydes à longue chaîne en C₆-C₃₀, de préférence encore tels que le rapport pondéral de l'éthylène-oxyde et/ou de propylène-oxyde avec alkylènes-oxydes dans le copolymère est de 5:95 à 70:30. Dans cette famille, on citera notamment les copolymères tels que les alkylènes-oxydes à longue chaîne sont disposés en blocs ayant un poids moléculaire moyen de 1.000 à 10.000, par exemple un copolymère bloc de polyoxyethylène/polydodécyle glycol tel que les éthers de dodécanediol (22 mol) et de polyéthylène glycol (45 OE) commercialisés sous la marque ELFACOS ST9 par Akzo Nobel.

Parmi les pâteux esters, on préfère notamment :
- les esters d'un glycérol oligomère, notamment les esters de diglycérol, en particulier les condensats d'acide adipique et de glycérol, pour lesquels une partie des groupes hydroxyle des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol
- le propionate d'arachidyle commercialisé sous la marque Waxenol 801 par Alzo,
- les esters de phytostérol,
- les polyesters non réticulés résultant de la polycondensation entre un diacide ou un polyacide carboxylique linéaire ou ramifié en C₄-C₅₀ et un diol ou un polyol en C₂-C₅₀, différent du polyester décrit précédemment,
- les esters aliphatiques d'ester résultant de l'estérification d'un ester d'acide hydroxycarboxylique aliphatique par un acide monocarboxylique aliphatique; et leurs mélanges, comme :
   - l'ester résultant de la réaction d'estérification de l'huile de ricin hydrogénée avec l'acide isostéarique dans les proportions 1 pour 1 (1/1) ou monoisostéarate d'huile de ricin hydrogénée,
   - l'ester résultant de la réaction d'estérification de l'huile de ricin hydrogénée avec l'acide isostéarique dans les proportions 1 pour 2 (1/2) ou le diisostéarate d'huile de ricin hydrogénée,
   - l'ester résultant de la réaction d'estérification de l'huile de ricin hydrogénée avec l'acide isostéarique dans les proportions 1 pour 3 (1/3) ou triisostéarate d'huile de ricin hydrogénée,
   - et leurs mélanges.

Parmi les composés pâteux d'origine végétale, on choisira de préférence un mélange de stérols de soja et de pentaérythritol oxyéthyléné (5OE) oxypropyléné (5 OP), commercialisé sous la référence Lanolide par la société VEVY.

Le composé pâteux représente de préférence 1 à 99 %, mieux 1 à 60 %, mieux 2 à 30 % et mieux encore 5 à 20 % en poids de la composition.

Comme précisée précédemment, les compositions selon l'invention peuvent comprendre également une ou plusieurs charges, notamment en une teneur allant de 0,01 % à 50 % en poids, par rapport au poids total de la composition, de préférence allant de 0,01 % à 30 % en poids.

Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues. On peut citer le talc, le mica, la silice, le kaolin, les poudres de polyamide (Nylon^{®}) (Orgasol^{®} de chez Atochem), de poly-β-alanine et de polyéthylène, les poudres de polymères de tétrafluoroéthylène (Téflon^{®}), la lauroyl-lysine, l'amidon, le nitrure de bore, les microsphères creuses polymériques telles que celles de chlorure de polyvinylidène/acrylonitrile comme l'Expancel^{®} (Nobel Industrie), de copolymères d'acide acrylique (Polytrap^{®} 603 de la société Dow Corning) et les microbilles de résine de silicone (Tospearls^{®} de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads^{®} de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, le Polypore^{®} L 200 (Chemdal Corporation). On peut encore citer les charges à base de silice comme l'Aerosil 200, l'Aerosil 300 ; le Sunsphare L-31, le Sunphare H-31 commercialisés par Asahi Glass ; le Chemicelen commercialisé par Asahi Chemical ; les composites de silice et de dioxyde de titane comme la série TSG commercialisée par Nippon Sheet Glass. Enfin, on peut citer les poudres de poyuréthanne, en particulier les poudres de polyuréthanne réticulé comprenant un copolymère, ledit copolymère comprenant du triméthylol hexyllactone. En particulier, il peut s'agir d'un polymère d'hexaméthylène di-isocyanate/triméthylol hexyllactone. De telles particules sont notamment disponibles dans le commerce, par exemple sous la dénomination de PLASTIC POWDER D-400^{®} ou PLASTIC POWDER D-800^{®} de la société TOSHIKI.

### MATIERE COLORANTE

Avantageusement, les compositions de l'invention comprennent au moins une matière colorante qui peut être choisie parmi les colorants, les pigments, les nacres et leurs mélanges. Cette matière colorante peut représenter de 0,001 à 98 %, de préférence de 0,5 à 85 % et mieux de 1 à 60 % du poids total de la composition.

Pour des raisons évidentes, ces matières colorantes sont mises en oeuvre dans les compositions selon l'invention, de manière à ne pas porter préjudice à l'effet plus particulièrement recherché selon l'invention et qui vise notamment à procurer un effet de maquillage naturel.

Toutefois, il peut être avantageux pour les matières colorantes mises en oeuvre de procurer un effet de couleur autre que blanc, ou encore de ne pas être transparentes.

Pour une composition sous forme de pâte ou coulée telle que les rouges à lèvres ou les produits de maquillage du corps, on utilise en général de 0,5 à 50 % de matière colorante, de préférence de 2 à 40 % et mieux de 5 à 30 %, par rapport au poids total de la composition.

Les colorants sont de préférence des colorants liposolubles, bien que les colorants hydrosolubles puissent être utilisés. Les colorants liposolubles sont par exemple le rouge Soudan, le D & C Red 17, le D & C Green 6, le β-carotène, l'huile de soja, le brun Soudan, le D & C Yellow 11, le D & C Violet 2, le D & C orange 5, le jaune quinoléine, le rocou. Ils peuvent représenter de 0 à 20 % du poids de la composition et mieux de 0,1 à 6 %. Les colorants hydrosolubles sont notamment le jus de betterave, le bleu de méthylène et peuvent représenter de 0,1 à 6 % en poids de la composition (si présents).

Par pigments, il faut comprendre des particules blanches ou colorées, minérales ou organiques, insolubles dans la phase grasse liquide, destinées à colorer et/ou opacifier la composition. Par nacres, il faut comprendre des particules irisées, notamment produites par certains mollusques dans leur coquille ou bien synthétisées.

Les pigments peuvent être présents dans la composition à raison de 0,05 à 30 % du poids de la composition finale, et de préférence à raison de 2 à 20 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium (D & C Red N°7), aluminium.

Les nacres peuvent être présentes dans la composition à raison de 0,001 à 20 % du poids total de la composition, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.

Les compositions peuvent également contenir avantageusement des pigments gonio-chromatiques, par exemple des pigments multicouches interférentiels, et/ou des pigments réfléchissants. Ces deux types de pigments sont décrits dans le document FR 0 209 246.

### COMPOSANTS ANNEXES

Selon un mode de réalisation avantageux, les composés considérés selon l'invention peuvent être associés dans les compositions selon l'invention à d'autres actifs susceptibles de participer à la manifestation de l'effet recherché, à savoir un effet bonne mine au niveau de la peau et/ou des lèvres, ou un effet repulpeur au niveau des lèvres.

Cet actif peut par exemple favoriser la microcirculation sanguine au niveau de la matière kératinique contactée.

Il peut ainsi s'agir d'un composé choisi parmi :
- des agents favorisant la production de monoxyde d'azote ;
- des agents anti-hypertenseurs ; en particulier des ouvreurs de canaux potassium ;
- des agents inhibiteurs de phosphodiestérases ;
- des flavonoïdes ou des flavoglycosides ;
- des glucosides ;
- des extraits végétaux aux propriétés vasodilatatrices ;
- des peptides vasodilatateurs non donneurs de NO ;
- d'autres agents vasodilatateurs ; et
- des agents modulateurs de la température.

Les compositions selon l'invention peuvent également contenir des ingrédients couramment utilisés en cosmétique, tels que les vitamines, les oligo-éléments, les adoucissants, les séquestrants, les parfums, les agents alcalinisants ou acidifiants, les conservateurs, les filtres solaires, les tensioactifs, les polymères filmogènes, les antioxydants, les agents propulseurs, ou leurs mélanges.

Bien entendu, l'homme du métier veillera à choisir ce ou ces éventuels composés complémentaires, et/ou leur quantité, de manière telle que les propriétés avantageuses de la composition correspondante selon l'invention ne soient pas, ou substantiellement pas, altérées par l'adjonction envisagée.

Les compositions selon l'invention peuvent être fabriquées par les procédés connus, généralement utilisés dans le domaine cosmétique ou dermatologique. En particulier, elles peuvent être obtenues par chauffage des différents constituants à la température de fusion des cires les plus élevées, puis coulage du mélange fondu dans un moule (coupelle ou doigt de gant). Elles peuvent aussi être obtenues par extrusion comme décrit dans la demande EP-A-667 146.

La composition de l'invention peut se présenter sous la forme solide, pâteuse ou liquide plus ou moins fluide. Elle sera préférentiellement un gloss ou une pâte souple de soin ou de maquillage des lèvres.

L'invention est illustrée plus en détail dans les exemples suivants. Les pourcentages sont donnés en poids.

### EXEMPLES

### Formulations de glos s

| **Exemple 1** | |
|---|---|
| Composants | Concentration (% massique) |
| Polybutène PM : 920 | 29,95 |
| Polybutène PM : 1290 | 34,00 |
| Isononanoate d'isononyle | 2,89 |
| octyl-2 dodecanol | 9,39 |
| tri-mellitate de tri-decyle | 13,14 |
| Caprylate de glyceryle ¹ | 5,00 |
| Silice pyrogénée hydrophobe, traitée en surface par di-methylsilane | 5,00 |
| Parfum | 0,5 |
| saccharinate de sodium | 0,12 |

| | |
|---|---|
| ¹ CAPMUL 708G - ABITEC | |

| | Concentration (% massique) | | |
|---|---|---|---|
| Composants | Exemple 2 | Exemple 3 | Exemple 4 |
| Isononanoate d'isononyle | 4 | 4 | 4 |
| Caprylate/caprate de glyceryle ² | 1,5 | 0 | 1.5 |
| Octyl-2 dodecanol | 10 | 10 | 10 |
| Mélange de P-hydroxybenzoate, isobutyle, N-butyle (40/30/30) | 0,4 | 0,4 | 0,4 |
| Polybutène (PM : 2060) | 4 | 4 | 4 |
| Caprylyl glycol ³ | 0 | 1 | 0,5 |
| Polybutène PM : 1290 | 30 | 30 | 30 |
| Polybutène PM : 920) | 29,75 | 30,25 | 29,25 |
| Tétra-iso-stéarate de pentaerythrityle (QS) | 20,35 | 20,35 | 20,35 |
| Total : | 100 | 100 | 100 |

| | | | |
|---|---|---|---|
| ² CAPMUL MCM ABITEC ³ DERMOSOFT OCTIOL STRAETMANS | | | |

Après application de chacun des gloss précédents sur les lèvres, il est constaté un gonflement des lèvres, procurant un effet repulpeur ou « plump » notable perceptible par l'utilisatrice.

## Revendications

1. Utilisation cosmétique d'au moins un glycol de chaîne hydrocarbonée en C₄-C₁₆ et/ou d'un ester hydroxylé résultant de l'estérification de polyol et d'acide(s) carboxylique(s) en C₄ à C₁₆ comme agent destiné à renforcer la carnation naturelle de la peau et/ou des lèvres et/ou le volume naturel des lèvres dans une composition de soin et/ou de maquillage de la peau et/ou des lèvres.

2. Utilisation selon la revendication 1, dans laquelle ledit agent est destiné à conférer à la peau notamment du visage un effet bonne mine.

3. Utilisation selon la revendication 1 ou 2, dans laquelle ledit agent est destiné à homogénéiser et/ou clarifier le teint et/ou diminuer l'effet de teint brouillé.

4. Utilisation selon la revendication 1, dans laquelle ledit agent est destiné à augmenter la taille et/ou le volume des lèvres et/ou à les modeler et/ou à les rendre plus lisses.

5. Utilisation selon l'une quelconque des revendications précédentes, dans laquelle ledit agent est destiné à stimuler la coloration naturelle des lèvres.

6. Utilisation selon l'une quelconque des revendications précédentes, dans laquelle ledit agent est formé d'au moins un ester hydroxylé résultant de l'estérification de polyol et d'acide(s) carboxylique(s) en C₄ à C₁₆ et d'un glycol de chaîne hydrocarbonée en C₄-C₁₆.

7. Utilisation selon l'une quelconque des revendications 1 à 5, dans laquelle ledit agent est formé d'un ester hydroxylé résultant de l'estérification de polyol et d'acide(s) carboxylique(s) en C₄ à C₁₆.

8. Utilisation selon l'une quelconque des revendications précédentes dans laquelle ledit ester est un mono-ester hydroxylé, un di-ester hydroxylé ou un de leurs mélanges.

9. Utilisation selon l'une quelconque des revendications précédentes dans laquelle l'ester est en C₁₀ à C₂₀ et possède au moins une chaîne grasse.

10. Utilisation selon l'une quelconque des revendications précédentes, dans laquelle ledit ester porte au moins une et de préférence deux fonction(s) OH libre(s) au niveau de son résidu alcool.

11. Utilisation selon l'une quelconque des revendications précédentes, dans laquelle l'ester résulte de l'estérification d'un polyol qui est un dérivé hydrocarboné saturé ou insaturé, linéaire ou ramifié, en C₂ à C₁₅, ayant au moins deux, et en particulier au moins trois fonctions hydroxyles libres.

12. Utilisation selon l'une quelconque des revendications précédentes, dans laquelle l'ester résulte de l'estérification d'un polyol qui est un dérivé hydrocarboné en C₂ à C₈.

13. Utilisation selon l'une quelconque des revendications précédentes, dans laquelle l'ester résulte de l'estérification d'un polyol qui est choisi parmi le propylène glycol et le glycérol.

14. Utilisation selon l'une quelconque des revendications précédentes, dans laquelle l'ester résulte de l'estérification d'un acide qui est un dérivé hydrocarboné saturé ou insaturé, monocarboxylique en C₇ à C₁₀.

15. Utilisation selon l'une quelconque des revendications précédentes, dans laquelle l'ester résulte de l'estérification d'un acide qui est choisi parmi les acides heptanoïque, caprylique et caprique.

16. Utilisation selon l'une quelconque des revendications précédentes, dans laquelle ledit agent est un ester choisi parmi les caprylate de mono et /ou di-glycéryle, héptanoate de mono et/ou di glycéryle, caprylate de mono et/ou di glycéryle, caprylate de propylèneglycol, héptanoate de propylèneglycol et leurs mélanges.

17. Utilisation selon l'une quelconque des revendications précédentes, dans laquelle ledit agent comprend au moins un ester de caprylate de monoglycéryle.

18. Utilisation selon l'une quelconque des revendications 1 à 6 et 8 à 17, dans laquelle ledit agent est formé d'un glycol de chaîne hydrocarbonée en C₇-C₁₄.

19. Utilisation selon l'une quelconque des revendications 1 à 6 et 8 à 17, dans laquelle le glycol a une chaîne hydrocarbonée en C₆-C₁₂.

20. Utilisation selon l'une quelconque des revendications 1 à 6 et 8 à 19, dans laquelle le glycol a une chaîne hydrocarbonée en C₇-C₁₀.

21. Utilisation selon l'une quelconque des revendications 1 à 6 et 8 à 20, dans laquelle le glycol a une chaîne hydrocarbonée en C₈-C₁₀.

22. Utilisation selon l'une quelconque des revendications 1 à 6 et 8 à 21, dans laquelle le glycol est le caprylyl glycol.

23. Utilisation selon l'une quelconque des revendications précédentes, dans laquelle la composition est adaptée à une application topique.

24. Utilisation selon l'une quelconque des revendications précédentes, dans laquelle la composition est sous la forme d'une base de soin, d'une crème de soin, d'une base de maquillage, d'une crème teintée, d'un fond de teint, d'un rouge à lèvres, d'un gloss liquide, d'une pâte à lèvres, d'un crayon de contour des lèvres, d'un baume à lèvres ou d'un vernis à lèvres.

25. Produit de maquillage des lèvres contenant, dans un milieu physiologiquement acceptable, moins de 5 % en poids d'eau et au moins un glycol de chaîne hydrocarbonée en C₄-C₁₆ et comprenant de plus un ester hydroxylé de polyol et d'acide(s) carboxylique(s) en C₄-C₁₆.

26. Produit selon la revendication précédente, comprenant moins de 1 % en poids d'eau et en particulier anhydre.

27. Composition cosmétique destinée au soin et/ou au maquillage de la peau et/ou des lèvres contenant dans un milieu physiologiquement acceptable, au moins :
- une matière colorante, et
- au moins un glycol de chaîne hydrocarbonée en C₄-C₁₆. et au moins un ester hydroxylé de polyol et d'acide(s) carboxylique(s) en C₄-C₁₆.

28. Composition selon l'une quelconque des revendications 25 à 27, dans laquelle ledit ester est tel que défini en revendications 7 à 17.

29. Composition selon l'une quelconque des revendications 25 à 28, contenant de 0,05 à 20 %, notamment de 0,1 à 10 %, voire au moins 5 %, en particulier de 0,2 à 5 %, et plus particulièrement 0,5 à 2 % en poids dudit glycol ou desdits glycols par rapport au poids total de la composition.

30. Composition selon l'une quelconque des revendications 25 à 29, contenant au moins une phase solvant non aqueuse.

31. Composition selon l'une quelconque des revendications 25 à 30, comprenant au moins un agent épaississant choisi parmi les cires et les corps gras pâteux.

32. Composition selon l'une quelconque des revendications 25 à 31, contenant au moins une matière colorante choisie parmi les pigments, les nacres et les colorants.

33. Composition selon l'une quelconque des revendications 25 à 32, contenant au moins une charge.

34. Composition selon l'une quelconque des revendications 25 à 33, dans laquelle ledit glycol est tel que défini en revendications 18 à 22.

35. Composition selon l'une quelconque des revendications 25 à34, se présentant sous la forme d'un produit de maquillage des lèvres ou de la peau.

36. Procédé de maquillage de la peau et/ou des lèvres visant à donner un effet bonne mine au teint, mettant en oeuvre une composition telle que définie dans l'une quelconque des revendications 25 à 35.

37. Procédé de maquillage des lèvres comprenant l'application sur les lèvres d'une composition telle que définie dans l'une quelconque des revendications 25 à 33.

## Patentansprüche

1. Kosmetische Verwendung mindestens eines Glycols mit einer Kohlenwasserstoffkette mit 4 bis 16 Kohlenstoffatomen und/oder eines sich aus der Veresterung von Polyol und Carbonsäure(n) mit 4 bis 16 Kohlenstoffatomen ergebenden hydroxylierten Esters als Mittel, das zur Verstärkung der natürlichen Farbe der Haut und/oder der Lippen und/oder des natürlichen Volumens der Lippen bestimmt ist, in einer Zusammensetzung zur Pflege und/oder zum Schminken der Haut und/oder der Lippen.

2. Verwendung nach Anspruch 1, bei der das Mittel dazu bestimmt ist, der Haut insbesondere des Gesichts ein gutes Aussehen zu verleihen.

3. Verwendung nach Anspruch 1 oder 2, bei der das Mittel dazu bestimmt ist, den Teint zu homogenisieren und/oder zu klären und/oder das schlechte Aussehen des Teints zu verringern.

4. Verwendung nach Anspruch 1, bei der das Mittel dazu bestimmt ist, die Größe und/oder das Volumen der Lippen zu vergrößern und/oder sie zu modellieren und/oder sie glatter zu machen.

5. Verwendung nach einem der vorhergehenden Ansprüche, bei der das Mittel dazu bestimmt ist, die natürliche Färbung der Lippen zu stimulieren.

6. Verwendung nach einem der vorhergehenden Ansprüche, bei der das Mittel von mindestens einem sich aus der Veresterung von Polyol und Carbonsäure(n) mit 4 bis 16 Kohlenstoffatomen ergebenden hydroxylierten Ester und einem Glycol mit einer Kohlenwasserstoff kette mit 4 bis 16 Kohlenstoffatomen gebildet ist.

7. Verwendung nach einem der Ansprüche 1 bis 5, bei der das Mittel von einem hydroxylierten Ester gebildet ist, der sich aus der Veresterung von Polyol und Carbonsäure(n) mit 4 bis 16 Kohlenstoffatomen ergibt.

8. Verwendung nach einem der vorhergehenden Ansprüche, bei der der Ester ein hydroxylierter Monoester, ein hydroxylierter Diester oder eine ihrer Mischungen ist.

9. Verwendung nach einem der vorhergehenden Ansprüche, bei der der Ester 10 bis 20 Kohlenstoffatome aufweist und mindestens eine Fettkette besitzt.

10. Verwendung nach einem der vorhergehenden Ansprüche, bei der der Ester mindestens eine und vorzugsweise zwei freie OH-Funktionen auf Höhe seines Alkoholrests trägt.

11. Verwendung nach einem der vorhergehenden Ansprüche, bei der der Ester sich aus der Veresterung eines Polyols ergibt, das ein lineares oder verzweigtes, gesättigtes oder ungesättigtes Kohlenwasserstoffderivat mit 2 bis 15 Kohlenstoffatomen ist, das mindestens zwei und insbesondere mindestens drei freie Hydroxylfunktionen aufweist.

12. Verwendung nach einem der vorhergehenden Ansprüche, bei der der Ester sich aus der Veresterung eines Polyols ergibt, das ein Kohlenwasserstoffderivat mit 2 bis 8 Kohlenstoffatomen ist.

13. Verwendung nach einem der vorhergehenden Ansprüche, bei der der Ester sich aus der Veresterung eines Polyols ergibt, das aus Propylenglycol und Glycerin ausgewählt ist.

14. Verwendung nach einem der vorhergehenden Ansprüche, bei der der Ester sich aus der Veresterung einer Säure ergibt, die ein gesättigtes oder ungesättigtes monocarboxylisches Kohlenwasserstoffderivat mit 7 bis 10 Kohlenstoffatomen ist.

15. Verwendung nach einem der vorhergehenden Ansprüche, bei der der Ester sich aus der Veresterung einer Säure ergibt, die aus Heptansäure, Caprylsäure und Caprinsäure ausgewählt ist.

16. Verwendung nach einem der vorhergehenden Ansprüche, bei der das Mittel ein Ester ist, der ausgewählt ist aus Mono- und/oder Diglycerylcaprylat, Mono- und/oder Diglycerylheptanoat, Mono- und/oder Diglycerylcaprylat, Propylenglycolcaprylat, Propylenglycolheptanoat und ihren Mischungen.

17. Verwendung nach einem der vorhergehenden Ansprüche, bei der das Mittel mindestens einen Monoglycerylcaprylatester umfasst.

18. Verwendung nach einem der Ansprüche 1 bis 6 und 8 bis 17, bei der das Mittel von einem Glycol mit einer Kohlenwasserstoffkette mit 7 bis 14 Kohlenstoffatomen gebildet ist.

19. Verwendung nach einem der Ansprüche 1 bis 6 und 8 bis 17, bei der das Glycol eine Kohlenwasserstoffkette mit 6 bis 12 Kohlenstoffatomen aufweist.

20. Verwendung nach einem der Ansprüche 1 bis 16 und 8 bis 19, bei der das Glycol eine Kohlenwasserstoffkette mit 7 bis 10 Kohlenstoffatomen aufweist.

21. Verwendung nach einem der Ansprüche 1 bis 6 und 8 bis 20, bei der das Glycol eine Kohlenwasserstoffkette mit 8 bis 10 Kohlenstoffatomen aufweist.

22. Verwendung nach einem der Ansprüche 1 bis 6 und 8 bis 21, bei der das Glycol Caprylylglycol ist.

23. Verwendung nach einem der vorhergehenden Ansprüche, bei der die Zusammensetzung an eine topische Anwendung angepasst ist.

24. Verwendung nach einem der vorhergehenden Ansprüche, bei der die Zusammensetzung in Form einer Pflegegrundlage, einer Pflegecreme, einer Schminkgrundlage, einer getönten Creme, einer Grundierung, eines Lippenrots, eines flüssigen Gloss, einer Lippenpaste, eines Lippeniconturenstifts, eines Lippenbalsams oder eines Nagellacks ist.

25. Produkt zum Schminken der Lippen, das in einem physiologisch annehmbaren Medium weniger als 5 Gew.% Wasser und mindestens ein Glycol mit einer Kohlenwasserstoffkette mit 4 bis 16 Kohlenstoffatomen enthält und außerdem einen hydroxylierten Ester von Polyol und Carbonsäure(n) mit 4 bis 16 Kohlenstoffatomen umfasst.

26. Produkt nach dem vorhergehenden Anspruch, das weniger als 1 Gew.-% Wasser umfasst und insbesondere wasserfrei ist.

27. Kosmetische Zusammensetzung, die zur Pflege und/oder zum Schminken der Haut und/oder der Lippen bestimmt ist und in einem physiologisch annehmbaren Medium mindestens umfasst:
- ein Färbungsmaterial und
- mindestens ein Glycol mit einer Kohlenwasserstoffkette mit 4 bis 16 Kohlenstoffatomen und mindestens einen hydroxylierten Ester von Polyol und Carbonsäure(n) mit 4 bis 16 Kohlenstoffatomen.

28. Zusammensetzung nach einem der Ansprüche 25 bis 27, bei der der Ester so ist, wie er in den Ansprüche 7 bis 17 definiert ist.

29. Zusammensetzung nach einem der Ansprüche 25 bis 28, die 0,05 bis 20 Gew.-%, insbesondere 0,1 bis 10 Gew.-% oder mindestens 5 Ges.-%, insbesondere 0,2 bis 5 Gew.-% und im Besonderen 0,5 bis 2 Gew.-% Glycol oder Glycole bezüglich des Gesamtgewichts der Zusammensetzung enthält.

30. Zusammensetzung nach einem der Ansprüche 25 bis 29, die mindestens eine nicht wässrige Lösungsmittelphase enthält.

31. Zusammensetzung nach einem der Ansprüche 25 bis 30, die mindestens ein Verdickungsmittel umfasst, das aus den Wachsen und den pastenartigen Fetten ausgewählt ist.

32. Zusammensetzung nach einem der Ansprüche 25 bis 31, die mindestens ein Färbungsmaterial enthält, das aus Pigmenten, Perlmuttstoffen und Farbstoffen ausgewählt ist.

33. Zusammensetzung nach einem der Ansprüche 25 bis 32, die mindestens einen Füllstoff enthält.

34. Zusammensetzung nach einem der Ansprüche 25 bis 33, bei der das Glycol so ist, wie in den Ansprüchen 18 bis 22 definiert ist.

35. Zusammensetzung nach einem der Ansprüche 25 bis 34, die in Form eines Produkts zum Schminken der Lippen oder der Haut vorliegt.

36. Verfahren zum Schminken der Haut und/oder der Lippen, das dazu dient, dem Teint ein gutes Aussehen zu verleihen, und eine Zusammensetzung, wie sie in einem der Ansprüche 25 bis 35 definiert ist, verwendet.

37. Verfahren zum Schminken der Lippen, das das Auftragen einer Zusammensetzung, wie sie in einem der Ansprüche 25 bis 33 definiert ist, auf die Lippen umfasst.

## Claims

1. Cosmetic use of at least one glycol with a C₄-C₁₆ hydrocarbon-based chain and/or of a hydroxylated ester resulting from the esterification of polyol and of C₄-C₁₆ carboxylic acid(s), as an agent for reinforcing the natural flesh tint of the skin and/or of the lips and/or the natural volume of the lips in a composition for caring for and/or making up the skin and/or the lips.

2. Use according to Claim 1, in which the said agent is intended for giving the skin, especially of the face, a healthy complexion effect.

3. Use according to Claim 1 or 2, in which the said agent is intended for homogenizing and/or clarifying the complexion and/or for reducing the hazy complexion effect.

4. Use according to Claim 1, in which the said agent is intended for increasing the size and/or volume of the lips and/or for modelling them and/or making them smoother.

5. Use according to any one of the preceding claims, in which the said agent is intended for stimulating the natural coloration of the lips.

6. Use according to any one of the preceding claims, in which the said agent is formed from at least one hydroxylated ester resulting from the esterification of polyol and of C₄-C₁₆ carboxylic acid(s) and of a glycol with a C₄-C₁₆ hydrocarbon-based chain.

7. Use according to any one of Claims 1 to 5, in which the said agent is formed from a hydroxylated ester resulting from the esterification of polyol and of C₄-C₁₆ carboxylic acid(s).

8. Use according to any one of the preceding claims, in which the said ester is a hydroxylated monster, a hydroxylated diester or a mixture thereof.

9. Use according to any one of the preceding claims, in which the ester is of C₁₀-C₂₀ and contains at least one fatty chain.

10. Use according to any one of the preceding claims, in which the said ester bears at least one and preferably two free OH function(s) on its alcohol residue.

11. Use according to any one of the preceding claims, in which the ester results from the esterification of a polyol which is a saturated or unsaturated, linear or branched C₂-C₁₅ hydrocarbon-based derivative containing at least two and in particular at least three free hydroxyl functions.

12. Use according to any one of the preceding claims, in which the ester results from the esterification of a polyol which is a C₂-C₈ hydrocarbon-based derivative.

13. Use according to any one of the preceding claims, in which the ester results from the esterification of a polyol which is chosen from propylene glycol and glycerol.

14. Use according to any one of the preceding claims, in which the ester results from the esterification of an acid which is a saturated or unsaturated C₇-C₁₀ monocarboxylic hydrocarbon-based derivative.

15. Use according to any one of the preceding claims, in which the ester results from the esterification of an acid which is an ester chosen from heptanoic acid, caprylic acid and capric acid.

16. Use according to any one of the preceding claims, in which the said agent is an ester chosen from monoglyceryl and/or diglyceryl caprylate, monoglyceryl and/or diglyceryl heptanoate, monoglyceryl and/or diglyceryl caprylate, propylene glycol caprylate and propylene glycol heptanoate, and mixtures thereof.

17. Use according to any one of the preceding claims, in which the said agent comprises at least one ester of monoglyceryl caprylate.

18. Use according to any one of Claims 1 to 6 and 8 to 17, in which the said agent is formed from a glycol with a C₇-C₁₄ hydrocarbon-based chain.

19. Use according to any one of Claims 1 to 6 and 8 to 17, in which the glycol has a C₆-C₁₂ hydrocarbon-based chain.

20. Use according to any one of Claims 1 to 6 and 8 to 19, in which the glycol has a C₇-C₁₀ hydrocarbon-based chain.

21. Use according to any one of Claims 1 to 6 and 8 to 20, in which the glycol has a C₈-C₁₀ hydrocarbon-based chain.

22. Use according to any one of Claims 1 to 6 and 8 to 21, in which the glycol is caprylyl glycol.

23. Use according to any one of the preceding claims, in which the composition is suitable for topical application.

24. Use according to any one of the preceding claims, in which the composition is in the form of a care base, a care cream, a makeup base, a tinted cream, a foundation, a lipstick, a liquid gloss, a lip paste, a lip contour pencil, a lip balm or a lip varnish.

25. Lip makeup product containing, in a physiologically acceptable medium, less than 5% by weight of water and at least one glycol with a C₄-C₁₆ hydrocarbon-based chain and further comprising a hydroxylated ester of polyol and of C₄-C₁₆ carboxylic acid(s).

26. Product according to the preceding claim, comprising less than 1% by weight of water, and in particular being anhydrous.

27. Cosmetic composition for caring for and/or making up the skin and/or the lips, containing, in a physiologically acceptable medium, at least:
- one dyestuff, and
- at least one glycol with a C₄-C₁₆ hydrocarbon-based chain and at least a hydroxylated ester of polyol and of C₄-C₁₆ carboxylic acid(s).

28. Composition according to any one of Claims 25 to 27, in which the said ester is as defined in Claims 7 to 17.

29. Composition according to any one of Claims 25 to 28, containing from 0,05% to 20% especially from 0,1% to 10% or even at least 5%, in particular from 0.2% to 5% and more particularly 0.5% to 2% by weight of the said glycol or said glycols relative to the total weight of the composition.

30. Composition according to any one of Claims 25 to 29, containing at least one non-aqueous solvent phase.

31. Composition according to any one of Claims 25 to 30, comprising et lest one thickener chosen from waxes and pasty fatty subtances.

32. Composition according to any one of Claims 25 to 31, containing at least one dyestuff chosen from pigments, nacres and dyes.

33. Composition according to any one of Claims 25 to 32, containing at least one filler.

34. Composition according to any one of Claims 25 to 33, in which the said glycol is as defined in Claims 18 to 22.

35. Composition according to any one of Claims 25 to 34, which is in the form of a makeup product for the lips or the skin.

36. Process for making up the skin and/or the lips which is intended to give a healthy complexion effect, using a composition as defined in any one of Claims 25 to 35.

37. Process for making up the lips, comprising the application to the lips of a composition as defined in any one of Claims 25 to 33.
